# EUROPÄISCHE PATENTSCHRIFT

(11) **EP 3 772 659 B1**
(45) Veröffentlichungstag und Bekanntmachung des Hinweises auf die Patenterteilung: **27.09.2023**
(21) Anmeldenummer: 19190702.1
(22) Anmeldetag: 08.08.2019
(51) Int. Cl.: G01R 33/561, G01R 33/56, A61B 5/055

(54) **PARTIALVOLUMENANALYSE FÜR MAGNETRESONANZFINGERPRINTING**
PARTIAL VOLUME ANALYSIS FOR MAGNETIC RESONANCE FINGERPRINTING
ANALYSE DES VOLUMES PARTIELS DANS UN PROCÉDÉ D'EMPREINTE DE RÉSONANCE MAGNÉTIQUE

(43) Veröffentlichungstag der Anmeldung: 10.02.2021
(73) Patentinhaber: Siemens Healthcare GmbH, 91052 Erlangen (DE)
(72) Erfinder: Grodzki, David, 91058 Erlangen (DE)

(56) Entgegenhaltungen:
- ANAGHA DESHMANE ET AL.: "Accurate Synthetic FLAIR Images Using Partial Volume Corrected MR Fingerprinting", PROCEEDINGS OF THE INTERNATIONAL SOCIETY FOR MAGNETIC RESONANCE IN MEDICINE, ISMRM, 24TH ANNUAL MEETING AND EXHIBITION, SINGAPORE, 07 MAY - 13 MAY 2016, Nr. 1909, 22. April 2016 (2016-04-22), XP040682951,
- ZHENGWEI ZHOU ET AL.: "CEST Fingerprinting: Initial Study in Human Subjects", PROCEEDINGS OF THE INTERNATIONAL SOCIETY FOR MAGNETIC RESONANCE IN MEDICINE, ISMRM, JOINT ANNUAL MEETING ISMRM-ESMRMB, PARIS, FRANCE, 16-21 JUNE 2018, Nr. 417, 1. Juni 2018 (2018-06-01), XP040699625,
- DEBRA F. MCGIVNEY ET AL: "Magnetic resonance fingerprinting review part 2: Technique and directions", JOURNAL OF MAGNETIC RESONANCE IMAGING, 25. Juli 2019 (2019-07-25), XP055663708, US ISSN: 1053-1807, DOI: 10.1002/jmri.26877

## Beschreibung

Die Erfindung betrifft ein Verfahren zur quantitativen Magnetresonanzbildgebung, wobei wenigstens eine Zeitserie von Magnetresonanzbildern, die verschiedenen Zeitpunkten nach einer Anregung zugeordnet sind, eines Untersuchungsbereichs mit einer Magnetresonanzeinrichtung aufgenommen wird, wobei im Rahmen wenigstens einer Fingerprintingauswertung aus der Zeitserie für wenigstens einen Teil der Voxel der Magnetresonanzbilder ein zeitlicher Signalverlauf aus den Magnetresonanzdaten aller Magnetresonanzbilder ermittelt wird und zur Ermittlung wenigstens einer Ergebnisinformation der jeweilige Signalverlauf mit wenigstens einem aus einer Datenbank abgerufenen Vergleichsverlauf abgeglichen wird. Daneben betrifft die Erfindung eine Magnetresonanzeinrichtung, ein Computerprogramm und einen elektronisch lesbaren Datenträger.

Die Magnetresonanzbildgebung stellt ein wichtiges Werkzeug insbesondere in der Medizin, beispielsweise bei der Diagnose, dar. Dabei war es bislang gängig, qualitativ gewichtete Magnetresonanzbilder aufzunehmen, beispielsweise T1-gewichtete, T2-gewichtete und/oder protondichtegewichtete Bilder. Inzwischen wurde allerdings auch eine quantitative Magnetresonanzbildgebung vorgeschlagen, um als Ergebnis beispielsweise eine quantitative Messkarte zu erhalten, in der voxelweise Relaxationszeiten oder sonstige Materialparameter abgebildet werden, insbesondere auch ein beispielsweise aufgrund der Materialparameter ableitbares Material, das in dem Voxel vorliegt.

Ein bekanntes quantitatives Magnetresonanzbildgebungsverfahren ist das sogenannte Magnetresonanz-Fingerprinting (MRF), mit dem in einer Messung voxelweise mehrere physikalische Werte gleichzeitig gemessen werden können, beispielsweise die Relaxationszeiten T1 und T2. Hierbei werden bevorzugt mit einer Einzelschuss-Bildgebungstechnik eine große Zahl von Magnetresonanzbildern als Zeitserie nacheinander akquiriert, wobei sich über den Verlauf Messparameter wie die Repetitionszeit, Flipwinkel und dergleichen ändern, so dass abhängig von den physikalischen Eigenschaften in den durch die Voxel vermessenen Bereichen andere Signalverläufe über die Magnetresonanzbilder entstehen. Diese Signalverläufe, die auch als Fingerabdrücke bezeichnet werden, können durch Abgleich mit Vergleichsverläufen in Datenbanken einem n-Tupel physikalischer Ergebniswerte, beispielsweise Materialparameter, zugeordnet werden, insbesondere sogar einem Material in dem Voxel, das aufgrund der Ergebniswerte identifiziert wird.

Ein erhoffter Vorteil des Magnetresonanz-Fingerprintings ist eine gewisse Robustheit gegenüber kleineren Abweichungen beziehungsweise Fehlern im Fingerabdruck, also Signalverlauf. Diese Robustheit wird gemäß dem Stand der Technik beispielsweise zum Ausgleich von Unterabtastungsartefakten der einzelnen Magnetresonanzbilder genutzt und soll insgesamt dazu führen, dass Magnetresonanz-Fingerprinting gegenüber kleineren Bewegungen im aufzunehmenden Untersuchungsbereich insensitiv ist.

Neben diesen technischen Vorteilen bietet das Magnetresonanz-Fingerprinting zudem einen großen Vorteil in der Einfachheit der Bedienung, da für die Messung letztlich lediglich ein Start-Bedienelement und ein Stopp-Bedienelement benötigt werden. Die Reihenfolge und Art der Magnetresonanzbilder ist dann gemäß den in der Datenbank vorliegenden Vergleichsverläufen automatisch gewählt. Auf diese Weise ist das Magnetresonanz-Fingerprintingverfahren besonders attraktiv für Benutzer mit schlechter ausgebildetem Personal, die gegebenenfalls nach kostengünstigen Lösungen suchen und von Magnetresonanz-Fingerprinting angesprochen werden.

Forschungsbestrebungen bezüglich des Magnetresonanz-Fingerprintings haben inzwischen auch zu einem Verfahren geführt, mit dem eine sogenannte Subvoxelquantifizierung (bzw. Subpixelquantifizierung, je nach Nomenklatur der Bildelemente) möglich sein ist. Dabei wird nicht länger jedem Voxel ein quantitativer Ergebniswert zugeordnet, der dem die größte Übereinstimmung aufweisenden Vergleichsverlauf zugeordnet ist, sondern es kann eine Ergebniswertverteilung, beispielsweise eine Materialverteilung, für das Voxel angegeben werden. In einer solchen Subvoxelquantifizierung, wie sie beispielsweise in dem Artikel von Anagha Vishwas Desmane et al., "Validation of Tissue Characterization in Mixed Voxels using MR Fingerprinting", Proc. Intl. Soc. Mag. Reson. Med. 22 (2014), Seite 94, beschrieben ist, werden Vergleichsverläufe anhand erwarteter Ergebnisinformation, beispielsweise dort vermutete Materialien, manuell vorgegeben. Der relative Anteil jedes Vergleichsverlaufs und somit jedes Materials an dem gemessenen Signalverlauf wird bestimmt, indem der Signalverlauf als Zusammensetzung der Vergleichsverläufe mit bestimmten Gewichten angesetzt wird und nach den Gewichten gelöst wird, beispielsweise durch Pseudoinversen-Methoden. Der Abgleich ist in diesem Fall also so zu verstehen, dass der Signalverlauf als eine Überlagerung der einzelnen ausgewählten Vergleichsverläufe verstanden wird, wobei bestimmt wird, wie stark die einzelnen, ausgewählten Vergleichsverläufe beitragen. Im Fall der Bestimmung einer Materialzusammensetzung, in dem die Ergebniswerte Anteile jeweiliger Materialien sind, geben dann die Gewichte die relativen Anteile der entsprechenden Materialien, für die die Vergleichsverläufe bereitgestellt wurden, an, und können beispielsweise als Ergebniswerte der Ergebnisinformation dienen.

Bei bestimmten Anwendungen der Medizintechnik ist es von besonderem Interesse, einzelne Materialien innerhalb eines untersuchten Objektes zu bestimmen bzw. aufzuspüren. Denkbar wäre es beispielsweise, gezielt nach Stoffen oder Pathologien zu suchen, wenn deren physikalische Eigenschaften, beispielsweise Relaxationszeiten und dergleichen, bekannt sind. Beispielhafte Zielmaterialien können koaguliertes Blut und/oder verschiedene Tumorgewebe umfassen. Wünschenswert wäre es hierbei, einen schnellen Scan durchzuführen, ohne dass sonstige zweidimensional oder dreidimensional aufgelöste Schnittbilder erzeugt werden. Das bedeutet, in diesem Fall werden zweckmäßigerweise äußerst große Voxel gewählt, die im Extremfall auch das gesamte untersuchte Objekt bzw. den gesamten Untersuchungsbereich umfassen können. Dabei wäre es beispielsweise wünschenswert, derartige Untersuchungen am Kopf bzw. in größeren Untersuchungsbereichen im Kopf und/oder bei kleinen Volumina, beispielsweise bei Proben, durchzuführen.

Problematisch ist in diesem Fall jedoch, dass die Zielmaterialien bei derartigen Anwendungsfällen, insbesondere aufgrund der Größe der Voxel, nur in vergleichsweise geringen Konzentrationen auftreten, so dass ihr Anteil am Magnetresonanzsignal entsprechend vergleichsweise klein ist.

Zur Umsetzung derartiger Scans, bei denen bestimmte Zielmaterialien detektiert werden sollen, wurde bislang lediglich vorgeschlagen, Magnetresonanzspektroskopie zu betreiben, um entsprechend dem Zielmaterial zuordenbare Peaks im Frequenzspektrum zu identifizieren. Nachteilhafterweise wird mit der Resonanzfrequenz nur eine Eigenschaft des gesuchten Zielmaterials berücksichtigt, nicht jedoch seine sonstigen physikalischen Eigenschafen, beispielsweise die Relaxationszeiten.

Der Erfindung liegt daher die Aufgabe zugrunde, eine zielgerichtetere und insbesondere verlässlichere Möglichkeit anzugeben, um Zielmaterialien, die in interessierenden Voxeln in geringer Konzentration vorliegen, detektieren zu können.

Zur Lösung dieser Aufgabe sind erfindungsgemäß ein Verfahren, eine Magnetresonanzeinrichtung, ein Computerprogramm und ein elektronisch lesbarer Datenträger gemäß den unabhängigen Ansprüchen vorgesehen. Vorteilhafte Ausgestaltungen ergeben sich aus den Unteransprüchen.

Bei einem Verfahren der eingangs genannten Art ist erfindungsgemäß vorgesehen, dass zur Detektion eines Zielmaterials, für das der erwartete Anteil im Vergleich zu dem Anteil wenigstens eines anderen in wenigstens einem interessierenden Voxel der Magnetresonanzbilder angenommenen Voxelmaterials gering ist,
- eine erste Zeitserie von Magnetresonanzbildern ohne Unterdrückung des Signals des Zielmaterials und eine zweite Zeitserie mit Unterdrückung des Signals des Zielmaterials aufgenommen werden und
- für das wenigstens eine interessierende Voxel der Magnetresonanzbilder durch Vergleich der Zeitserien eine die Anwesenheit des Zielmaterials beschreibende Detektionsinformation aus wenigstens einer Ergebnisinformation wenigstens einer der wenigstens einen Fingerprintingauswertung ermittelt wird.

Die vorliegende Erfindung dreht sich mithin um ein Verfahren zum Auffinden von Zielmaterialien mit geringer Konzentration, wobei in zumindest einem Teil der Messung das zu suchende Zielmaterial mit bekannten physikalischen Eigenschaften, insbesondere Relaxationszeiten und/oder Frequenz-Offset, unterdrückt wird und aus der Korrelation der beiden Messteile, mithin der ersten Zeitserie und der zweiten Zeitserie, das zu suchende Zielmaterial extrahiert wird. Dabei ist unter einem geringen Anteil im Rahmen der vorliegenden Erfindung insbesondere ein deutlich geringerer Anteil zu verstehen, beispielsweise höchstens ein Zehntel des Anteils eines in dem interessierenden Voxel am meisten vorhandenen Voxelmaterials. Das bedeutet, es ist davon auszugehen, dass eine einfache Subvoxelquantifizierung, wie oben beschrieben, aufgrund des geringen Anteils des Zielmaterials am Signal basierend auf einer Zeitserie keine verlässlichen Informationen liefern würde. Im Rahmen der vorliegenden Erfindung besteht jedoch eine Vergleichsmöglichkeit, die auf besonders vorteilhafte Weise die Herleitung weiterer, verlässlicher Informationen bezüglich des Zielmaterials erlaubt.

Denn während es im Prinzip auch denkbar wäre, zwei "normale", qualitative, protonengewichtete Magnetresonanzbilder aufzunehmen, wobei in einem der beiden Magnetresonanzbilder das zu suchende Signal unterdrückt ist, um dann ein Subtraktionsergebnis zu betrachten, ist bei den erwarteten schwachen Signalen jedoch zu befürchten, dass diese bei einer einzigen Messung von zwei Magnetresonanzbildern im Rauschen verlorengehen, mithin nicht mehr verlässlich festgestellt werden können. Demgegenüber bietet der hier beschriebene Magnetresonanz-Fingerprinting-Ansatz deutliche Vorteile. So werden zum einen alle Magnetresonanzbilder des Magnetresonanz-Fingerprinting-Verlaufes, insbesondere mehr als 100 oder mehr als 1000, berücksichtigt, so dass die Statistik deutlich erhöht wird. Aus diesem Grund ist das Magnetresonanz-Fingerprinting-Verfahren besonders robust gegenüber geringer Signalausbeute bzw. auch artefaktbehafteten Magnetresonanzbildern, beispielsweise aufgrund von Bewegung.

Im Wesentlichen wird im Rahmen der vorliegenden Erfindung also vorgeschlagen, zwei bezüglich eines Zielmaterials unterschiedliche Magnetresonanz-Fingerprinting-Messungen durchzuführen, um zwei Zeitserien zu erhalten, und durch deren Korrelation das Zielmaterial mit geringer Konzentration und damit sehr geringem Magnetresonanzsignal erkennen zu können. Dadurch wird es ermöglicht, in einer einzelnen, aufgrund großer Voxel schnell durchzuführenden Messung bestimmte Zielmaterialien gezielt zu suchen, beispielsweise bestimmtes Tumorgewebe und/oder koaguliertes Blut. Ziel des erfindungsgemäßen Vorgehens sind mithin keine zweidimensionalen oder dreidimensionalen Schnittbilder, sondern lediglich Aussagen über das Zielmaterial, im einfachsten Fall, ob das Zielmaterial vorhanden ist oder nicht. Aufgrund der verwendeten Fingerprintingauswertung ist das Verfahren auch dann, wenn am Ende eine qualitative Information (Zielmaterial vorhanden oder nicht) als Detektionsinformation bestimmt wird, als ein quantitatives Verfahren aufzufassen.

Zusammenfassend ermöglicht es das erfindungsgemäße Vorgehen mithin, eine sehr schnelle Untersuchung für ein gezieltes Screening auf bestimmte Zielmaterialien durchzuführen.

Zur Unterdrückung des Magnetresonanzsignals des Zielmaterials bei einer verwendeten Magnetresonanzsequenz kann ein auf wenigstens eine physikalische Eigenschaft des Zielmaterials angepasstes, insbesondere das Signal des Zielmaterials sättigendes Unterdrückungsmodul verwendet werden. Das bedeutet, es wird gezielt das zu suchende Magnetresonanzsignal unterdrückt, so dass das Magnetresonanzsignal des Zielmaterials nur in der ersten Zeitserie von Magnetresonanzbildern nennenswert vorhanden sein sollte. Hierzu können beispielsweise spektrale und/oder T1-selektive Unterdrückungsmethoden verwendet werden, die auf die physikalischen Eigenschaften des Zielmaterials angepasst sind. Konkret kann beispielsweise vorgesehen sein, dass das Unterdrückungsmodul, welches einen Teil der Magnetresonanzsequenz für die Aufnahme der zweiten Zeitserie bildet, wenigstens einen Inversionspuls und/oder eine Binomialpulsfolge umfasst und/oder ein T2-selektives Präparationsmodul ist. Derartige Signalunterdrückungsmöglichkeiten sind im Stand der Technik bereits weitgehend bekannt und sollen daher hier nicht näher dargelegt werden.

Im Folgenden sollen zwei konkrete Ansätze zum Vergleich der Zeitserien beschrieben werden, die in einem bevorzugten Ausführungsbeispiel beide verwendet werden können.

So kann in einer ersten konkreten Ausführungsform der vorliegenden Erfindung vorgesehen sein, dass zum Vergleich der Zeitserien
- für beide Zeitserien eine Fingerprintingauswertung für die Anteile von Kandidatenmaterialien in dem wenigstens einen interessierenden Voxel als Ergebnisinformation durchgeführt wird, wobei die Kandidatenmaterialien das Zielmaterial und das wenigstens eine Voxelmaterial umfassen, wobei Vergleichsverläufe für alle Kandidatenmaterialien aus der Datenbank abgerufen werden und ihr Beitrag zu dem Signalverlauf als der jeweilige Anteil ermittelt wird,
- die Ergebnisinformationen der beiden Fingerprintingauswertungen miteinander zur Ermittlung der Detektionsinformation verglichen werden.

Mit anderen Worten wird vorgeschlagen, für beide Zeitserien eine Subvoxelquantifizierung für die Voxelmaterialien und das Zielmaterial durchzuführen. Das Zielmaterial wird dabei für beide Zeitserien mit in die erwarteten Materialien aufgenommen, wobei anschließend die Ergebnisinformationen entsprechend verglichen werden. Dabei kann vorgesehen sein, dass ein Vorhandensein des Zielmaterials in einem interessierenden Voxel detektiert wird, wenn die Ergebnisinformation der ersten Zeitserien dies um wenigstens einen Schwellwert mehr als die insbesondere das Zielmaterial nicht anzeigende Ergebnisinformation der zweiten Zeitserie anzeigt. Wird also bei der hier vorgeschlagenen Subvoxelquantifizierung das Zielmaterial mit hinreichender Genauigkeit nur im Messteil ohne Unterdrückung identifiziert, kann davon ausgegangen werden, dass das Zielmaterial in der Probe bzw. im untersuchten interessierenden Voxel enthalten ist. In einem konkreten Beispiel werden je nach untersuchter Körperregion des Patienten (bzw. Körperregion, aus der eine untersuchte Probe stammt) die zu erwartenden dominierenden Gewebetypen in den Voxeln als Voxelmaterialien vorgegeben, beispielsweise innerhalb des Kopfes graue und weiße Gehirnsubstanz (GM, WM) und Liquor (LQ). Nachdem in der Subvoxelquantifizierung das Zielmaterial auch bereits als möglicher Stoff mit aufgenommen ist, kann zweckmäßigerweise in der Gewichtung bereits ein deutlich geringerer Wert für das Zielmaterial vorgegeben werden. Mit anderen Worten kann vorgesehen sein, dass für das Zielmaterial als Startwert für den Beitrag bzw. Anteil ein niedrigerer Wert als für die Voxelmaterialien angesetzt wird.

Dabei sei an dieser Stelle noch angemerkt, dass die Bestimmung der Anteile für die verschiedenen Kandidatenmaterialien insbesondere in einem Optimierungsverfahren und/oder unter Verwendung einer Pseudoinversions-Methode erfolgen kann, wobei insbesondere auf den eingangs zitierten Artikel von Anagha Vishwas Deshmane et al. verwiesen sei.

In einer zweiten, konkreten Ausbildung des Vergleichs der Zeitserien, die zweckmäßig zusätzlich zu der ersten gezeigten Variante verwendet wird, kann vorgesehen sein, dass:
- ein Differenzsignal als Differenz der Signalverläufe des wenigstens einen interessierenden Voxels ermittelt wird,
- das Differenzsignal als auszuwertender Signalverlauf einer Fingerprintingauswertung unterzogen wird, in der der wenigstens eine Vergleichsverlauf als dem Zielmaterial zugeordnet aus der Datenbank (10) abgerufen wird und/oder physikalische Eigenschaften beschreibende Ergebniswerte bestimmt werden, und
- das Vorhandensein des Zielmaterials unter Verwendung des gemäß der Ergebnisinformation ermittelten Übereinstimmungsgrades des wenigstens einen Vergleichsverlaufs mit dem Differenzsignal und/oder der bestimmten physikalischen Eigenschaften mit den physikalischen Eigenschaften des Zielmaterials detektiert wird.

In dieser Ausgestaltung wird mithin vorgeschlagen, die beiden Fingerprintingverläufe voneinander zu subtrahieren, so dass das Resultat zumindest im Prinzip nur noch dem Fingerprintingverlauf des zu suchenden Zielmaterials entsprechen sollte. Das bedeutet aber, es lässt sich ein Fingerprinting-Datenbankabgleich dahingehend durchführen, dass gezielt die Vergleichsverläufe, die gemäß Datenbank die physikalischen Eigenschaften des Zielmaterials beschreiben, herangezogen werden. Das Differenzsignal als mutmaßlicher Fingerprintingverlauf des Zielmaterials wird mithin in dieser Ausgestaltung mit entsprechenden Vergleichsverläufen aus der Datenbank verglichen. Alternativ oder zusätzlich kann selbstverständlich auch eine "übliche" Fingerprintingauswertung durchgeführt werden, indem letztlich durch allgemeinen Datenbankabgleich mit den verschiedenen, unterschiedlichen Ergebniswerten zugeordneten Vergleichsverläufen der Datenbank die physikalischen Eigenschaften, die sich aus dem Differenzsignal ergeben, ermittelt werden, welche dann ebenso mit den physikalischen Eigenschaften des Zielmaterials verglichen werden können, um einen Übereinstimmungsgrad festzustellen. Die physikalischen Eigenschaften können entsprechen Relaxationszeiten und dergleichen sein.

Wie bereits erwähnt, werden beide hier konkret beschriebenen Ansätze bevorzugt kumulativ verwendet, wobei in diesem Zusammenhang vorgesehen sein kann, dass die Ergebnisse beider Vergleichsvorgänge zur gegenseitigen Plausibilisierung verwendet werden und/oder statistisch zusammengeführt werden, insbesondere unter stärkerer Gewichtung der Auswertung des Differenzsignals, nachdem letztere Variante sich als robuster erweist. Beispielsweise kann also gesagt werden, dass dann, wenn das Zielmaterial mit hinreichender Genauigkeit nur im Messteil ohne Unterdrückung identifiziert wird und die physikalischen Eigenschaften des Differenzsignals mit den erwarteten Werten des zu suchenden Zielmaterials übereinstimmen, davon ausgegangen werden kann, dass das zu suchende Zielmaterial in der Probe bzw. im interessierenden Voxel enthalten ist.

Wie bereits erwähnt, werden dabei im Rahmen der vorliegenden Erfindung zur Begünstigung einer schnellen Messung große Voxel verwendet, wobei es insbesondere auch möglich ist, insbesondere bei einer Probe, nur ein einziges Voxel für den gesamten Untersuchungsbereich zu verwenden. Konkret kann beispielsweise vorgesehen sein, dass eine Voxelgröße von größer als 5 cm³, insbesondere größer als 15 cm³, verwendet wird.

Die Detektionsinformation kann zweckmäßigerweise einen eine Anwesenheitswahrscheinlichkeit des Zielmaterials beschreibenden Wahrscheinlichkeitswert umfassend ermittelt werden. Das bedeutet, je nach beispielsweise den Übereinstimmungsgraden und/oder allgemeinen Vergleichsergebnissen kann eine Wahrscheinlichkeit in Prozent ermittelt werden, zu der das zu detektierende Zielmaterial im interessierenden Voxel bzw. auch im Untersuchungsbereich insgesamt vorhanden ist.

Bei dem Zielmaterial kann es sich insbesondere, wie bereits eingangs erläutert wurde, um ein Material eines Tumorgewebes oder koaguliertes Blut handeln. Selbstverständlich sind grundsätzlich auch andere Ansätze denkbar, bei denen Materialien detektiert werden können, die in kleineren Mengen in einem größeren interessierenden Voxel vorkommen können, beispielsweise auch Kontrast- und/oder Wirkmittel und dergleichen.

Neben dem Verfahren betrifft die vorliegende Erfindung auch eine Magnetresonanzeinrichtung, aufweisend eine zur Ausführung des erfindungsgemäßen Verfahrens ausgebildete Steuereinrichtung. Sämtliche Ausführungen bezüglich des erfindungsgemäßen Verfahrens lassen sich analog auf die erfindungsgemäße Magnetresonanzeinrichtung übertragen, so dass auch mit dieser die bereits genannten Vorteile erhalten werden können.

Die Steuereinrichtung kann dabei wenigstens einen Prozessor und/oder wenigstens ein Speichermittel enthalten. Durch diese Hardwarekomponenten können, insbesondere unter Ergänzung von passenden Softwarekomponenten, Funktionseinheiten der Steuereinrichtung realisiert werden, die die Ausführung des erfindungsgemäßen Verfahrens ermöglichen. Neben einer Sequenzeinheit, die grundsätzlich bekannt ist und den Aufnahmebetrieb der Magnetresonanzeinrichtung steuert, kann beispielsweise auch eine Auswertungseinheit vorgesehen sein, um die Detektionsinformation zu ermitteln, insbesondere unter Verwendung einer Fingerprintingeinheit. Zur Realisierung von optionalen Ausgestaltungen der vorliegenden Erfindung können entsprechende Funktionseinheiten zugefügt werden.

Ein erfindungsgemäßes Computerprogramm ist beispielsweise direkt in ein Speichermittel einer Steuereinrichtung einer Magnetresonanzeinrichtung ladbar und weist Programmmittel auf, um die Schritte eines erfindungsgemäßen Verfahrens auszuführen, wenn das Computerprogramm in der Steuereinrichtung der Magnetresonanzeinrichtung ausgeführt wird. Das Computerprogramm kann auf einem erfindungsgemäßen elektronisch lesbaren Datenträger gespeichert sein, welcher mithin darauf gespeicherte elektronisch lesbare Steuerinformationen umfasst, welche zumindest ein erfindungsgemäßes Computerprogramm umfassen und derart ausgestaltet sind, dass sie bei Verwendung des Datenträgers in einer Steuereinrichtung einer Magnetresonanzeinrichtung ein erfindungsgemäßes Verfahren durchführen. Bei dem erfindungsgemäßen Datenträger handelt es sich insbesondere um einen nichttransienten Datenträger, beispielsweise eine CD-ROM.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: einen Ablaufplan eines Ausführungsbeispiels des erfindungsgemäßen Verfahrens,
- Fig. 2: eine erfindungsgemäße Magnetresonanzeinrichtung, und
- Fig. 3: den funktionalen Aufbau einer Steuereinrichtung der erfindungsgemäßen Magnetresonanzeinrichtung.

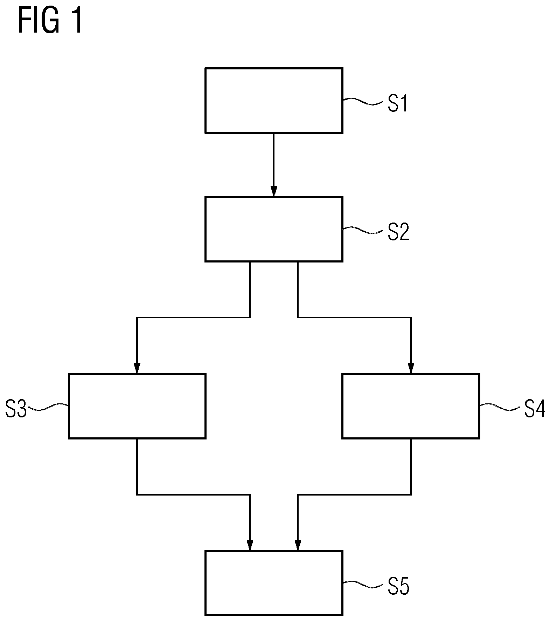

Fig. 1 zeigt einen Ablaufplan eines Ausführungsbeispiels des erfindungsgemäßen Verfahrens. Dabei soll unter Verwendung von Magnetresonanz-Fingerprinting überprüft werden, ob innerhalb eines Untersuchungsbereichs, beispielsweise eines Untersuchungsbereichs eines Patienten und/oder einer Probe, ein Zielmaterial vorhanden ist, welches in deutlich geringerer Konzentration in einem entsprechend interessierenden Voxel vorliegt als andere Voxelmaterialien in diesem Voxel. Beispielsweise kann es sich bei dem Zielmaterial um koaguliertes Blut oder ein Material eines Tumorgewebes handeln.

In einem Schritt S1 wird zunächst eine erste Zeitserie von Magnetresonanzbildern des Untersuchungsbereichs mit einer Magnetresonanzeinrichtung aufgenommen, wie dies beim Magnetresonanz-Fingerprinting üblich ist. Dabei ist keine besondere Anpassung der Magnetresonanzsequenz auf das Zielmaterial vorzunehmen.

In einem Schritt S2 wird eine zweite Zeitserie von Magnetresonanzbildern des Untersuchungsbereichs aufgenommen, wobei sich die Magnetresonanzbilder der zweiten Zeitserie von den Magnetresonanzbildern der ersten Zeitserie nur darin unterscheiden, dass die Magnetresonanzsequenz um ein Unterdrückungsmodul ergänzt wurde, welches das Magnetresonanzsignal des Zielmaterials unterdrückt, insbesondere sättigt. Dabei kann es sich beispielsweise um ein T1- und/oder T2-selektives Präparationsmodul handeln. Auch andere Möglichkeiten, um Magnetresonanzsignale bestimmter Materialien, hier des Zielmaterials, aufgrund der bekannten physikalischen Eigenschaften zu unterdrücken, sind im Stand der Technik bereits bekannt und können hier eingesetzt werden, beispielsweise Binomialpulsfolgen hinsichtlich der Larmorfrequenz.

In den Schritten S3 und S4 werden die beiden Zeitserien auf unterschiedliche Art und Weise in Auswertungsvorgängen miteinander verglichen. Zur Auswertung im Schritt S3 ist vorgesehen, für beide Zeitserien im Rahmen einer Fingerprintingauswertung eine Subvoxelquantifizierung vorzunehmen, wobei als Kandidatenmaterialien, die in dem jeweiligen interessierenden Voxel vorhanden sein können, sowohl das Zielmaterial als auch andere Voxelmaterialien vorgegeben werden. Beispielsweise kann im Fall des Gehirns als Untersuchungsbereich vorgesehen sein, als Voxelmaterialien weiße Gehirnmasse, graue Gehirnmasse und Liquor heranzuziehen. Für diese Kandidatenmaterialien werden aus einer Datenbank für die Fingerprintingauswertung jeweils passende Vergleichsverläufe abgerufen, die mit den aus den Magnetresonanzbildern für die interessierenden Voxel ermittelten Signalverläufen abgeglichen werden sollen, um Anteile des jeweiligen Kandidatenmaterials in dem interessierenden Voxel ermitteln zu können.

Konkret kann hierzu ein Ansatz vorgesehen sein, in dem angenommen wird, dass der Signalverlauf aus den unterschiedlichen Vergleichsverläufen mit einer entsprechenden Gewichtung, die dem Anteil des Kandidatenmaterials entspricht, zusammengesetzt ist. Zur Lösung nach den Gewichten, die unmittelbar die zu bestimmenden Anteile der Kandidatenmaterialien beschreiben, kann beispielsweise eine Pseudoinversen-Methode verwendet werden.

Die Ergebnisinformationen, im vorliegenden Fall die Anteile der verschiedenen Kandidatenmaterialien, aus den beiden Zeitserien werden ebenso noch im Schritt S3 miteinander verglichen, wobei beispielsweise dann, wenn die Ergebnisinformation der ersten Zeitserie das Vorhandensein des Zielmaterials anzeigt, und zwar um wenigstens einen Schwellwert mehr als die Ergebnisinformationen der zweiten Zeitserie, die idealerweise kein Vorhandensein des Zielmaterials anzeigt, davon ausgegangen werden kann, dass das Zielmaterial tatsächlich in dem betrachteten interessierenden Voxel vorhanden ist.

Im Schritt S4 wird unabhängig vom Schritt S3 anders vorgegangen, um die Zeitserien zu vergleichen. Dort werden zunächst die Zeitserien, genauer gesagt deren Signalverläufe, zur Bildung eines Differenzsignals voneinander subtrahiert. Dabei wird angenommen, dass das Differenzsignal nur noch den Fingerprintingverlauf des Zielmaterials widerspiegeln sollte, falls das Zielmaterial im interessierenden Voxel vorhanden ist. Entsprechend wird nun für das Differenzsignal eine übliche Fingerprintingauswertung durchgeführt, wobei entweder gezielt nur Vergleichsverläufe, die dem Zielmaterial zugeordnet sind, mithin dessen physikalische Eigenschaften beschreiben, zum Vergleich aus der Datenbank abgerufen werden, um einen Übereinstimmungsgrad zu ermitteln, und/oder wie beim Magnetresonanz-Fingerprinting üblich durch Datenbankabgleich physikalische Eigenschaften als Ergebniswerte bestimmt werden, die dann mit den entsprechenden physikalischen Eigenschaften des Zielmaterials verglichen werden können, um wiederum einen Übereinstimmungsgrad feststellen zu können. Stimmen die physikalischen Eigenschaften, die durch das Differenzsignal beschrieben werden, mit den erwarteten Werten für das Zielmaterial überein, kann davon ausgegangen werden, dass das Zielmaterial in der Probe bzw. im betrachteten interessierenden Voxel enthalten ist.

In einem Schritt S5 werden die Auswertungsergebnisse der Schritte S3 und S4 zur gegenseitigen Plausibilisierung verwendet und zudem zusammengeführt. Als letztendliche Detektionsinformation wird in diesem Ausführungsbeispiel eine Wahrscheinlich in Prozent dafür ausgegeben, dass sich das Zielmaterial in einem interessierenden Voxel/dem gesamten Untersuchungsbereich befindet. Es ist jedoch auch eine rein binäre Informationsausgebe denkbar (vorhanden/nicht vorhanden).

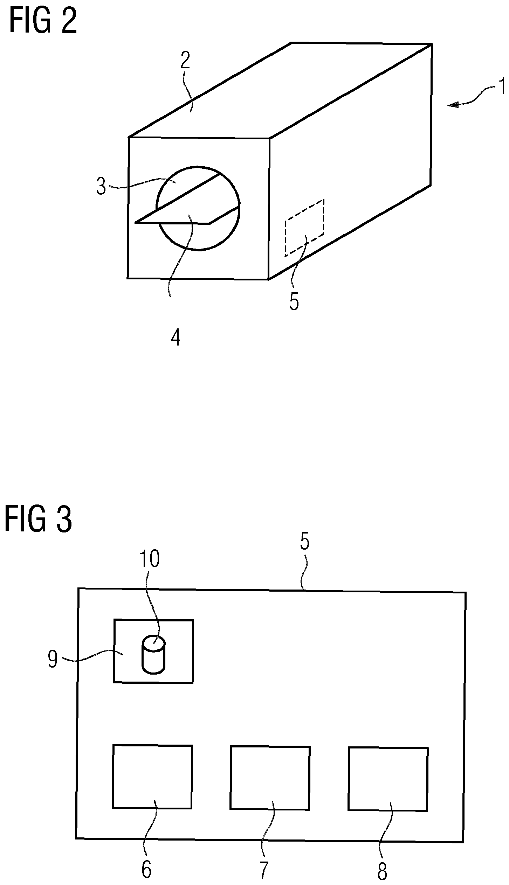

Fig. 2 zeigt eine Prinzipskizze einer erfindungsgemäßen Magnetresonanzeinrichtung 1. Diese weist, wie grundsätzlich bekannt, eine Hauptmagneteinheit 2 auf, in der eine Patientenaufnahme 3 ausgebildet ist. In die Patientenaufnahme 3 kann mittels einer Patientenliege 4 ein Patient (oder auch eine Probe) in den Bildgebungsbereich eingefahren werden. Die Patientenaufnahme 3 umgebend können eine Hochfrequenzspulenanordnung und eine Gradientenspulenanordnung der Magnetresonanzeinrichtung 1 (hier nicht näher gezeigt) vorgesehen sein. Der Betrieb der Magnetresonanzeinrichtung 1 wird durch eine Steuereinrichtung 5 gesteuert, die zur Durchführung des erfindungsgemäßen Verfahrens ausgebildet ist.

Der Aufbau der Steuereinrichtung 5 soll anhand von Fig. 3 näher erläutert werden. Demnach weist die Steuereinrichtung 5 zunächst, wie grundsätzlich bekannt, eine Sequenzeinheit 6 auf, über die die Komponenten der Magnetresonanzeinrichtung 1 zur Aufnahme von Magnetresonanzbildern angesteuert werden können. Das bedeutet, über die Sequenzeinheit 6 können insbesondere auch die Schritte S1 und S2 des erfindungsgemäßen Verfahrens durchgeführt werden.

Eine Auswerteeinheit 7 steuert die grundsätzliche Auswertung im Sinne der Schritte S3, S4 und S5, wobei zur Durchführung der Fingerprintingauswertungsvorgänge in den Schritten S3 und S4 auch gezielt eine Fingerprintingeinheit 8 vorgesehen sein kann, die auf die in einem Speichermittel 9 der Steuereinrichtung 5 gespeicherte Datenbank 10 mit den Vergleichsverläufen zugreifen kann.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen, welcher durch die Ansprüche definiert ist.

## Patentansprüche

1. Verfahren zur quantitativen Magnetresonanzbildgebung, wobei wenigstens eine Zeitserie von Magnetresonanzbildern, die verschiedenen Zeitpunkten nach einer Anregung zugeordnet sind, eines Untersuchungsbereichs mit einer Magnetresonanzeinrichtung (1) aufgenommen wird, wobei im Rahmen wenigstens einer Fingerprintingauswertung aus der Zeitserie für wenigstens einen Teil der Voxel der Magnetresonanzbilder ein zeitlicher Signalverlauf aus den Magnetresonanzdaten aller Magnetresonanzbilder ermittelt wird und zur Ermittlung wenigstens einer Ergebnisinformation der jeweilige Signalverlauf mit wenigstens einem aus einer Datenbank (10) abgerufenen Vergleichsverlauf abgeglichen wird,
**dadurch gekennzeichnet,**
**dass** zur Detektion eines Zielmaterials, für das der erwartete Anteil im Vergleich zu dem Anteil wenigstens eines anderen in wenigstens einem interessierenden Voxel der Magnetresonanzbilder angenommenen Voxelmaterials gering ist,
- eine die erste Zeitserie von Magnetresonanzbildern ohne Unterdrückung des Signals des Zielmaterials und eine zweite Zeitserie mit Unterdrückung des Signals des Zielmaterials aufgenommen werden und
- für das wenigstens eine interessierende Voxel der Magnetresonanzbilder durch Vergleich der Zeitserien eine die Anwesenheit des Zielmaterials beschreibende Detektionsinformation aus wenigstens einer Ergebnisinformation wenigstens einer der wenigstens einen Fingerprintingauswertung ermittelt wird.

2. Verfahren nach Anspruch 1, **dadurch gekennzeichnet, dass** zur Unterdrückung des Signals des Zielmaterials einer verwendeten Magnetresonanzsequenz ein auf wenigstens eine physikalische Eigenschaft des Zielmaterials angepasstes, insbesondere das Signal des Zielmaterials sättigendes Unterdrückungsmodul verwendet wird.

3. Verfahren nach Anspruch 2, **dadurch gekennzeichnet, dass** das Unterdrückungsmodul wenigstens einen Inversionspuls und/oder eine Binomialpulsfolge umfasst und/oder ein T2-selektives Präparationsmodul ist.

4. Verfahren nach einem der vorangehenden Ansprüche, **dadurch gekennzeichnet, dass** zum Vergleich der Zeitserien
- für beide Zeitserien eine Fingerprintingauswertung für die Anteile von Kandidatenmaterialien in dem wenigstens einen interessierenden Voxel als Ergebnisinformation durchgeführt wird, wobei die Kandidatenmaterialien das Zielmaterial und das wenigstens eine Voxelmaterial umfassen, wobei Vergleichsverläufe für alle Kandidatenmaterialien aus der Datenbank (10) abgerufen werden und ihr Beitrag zu dem Signalverlauf als der jeweilige Anteil ermittelt wird,
- die Ergebnisinformationen der beiden Fingerprintingauswertungen miteinander zur Ermittlung der Detektionsinformation verglichen werden.

5. Verfahren nach Anspruch 4, **dadurch gekennzeichnet, dass** im Rahmen der Fingerprintingauswertung eine Subvoxelquantifizierung vorgenommen wird, und für das Zielmaterial als Startwert für den Beitrag ein niedrigerer Wert als für die Voxelmaterialien angesetzt wird.

6. Verfahren nach einem der vorangehenden Ansprüche, **dadurch gekennzeichnet, dass** zum Vergleich der Zeitserien:
- ein Differenzsignal als Differenz der Signalverläufe des wenigstens einen interessierenden Voxels ermittelt wird,
- das Differenzsignal als auszuwertender Signalverlauf einer Fingerprintingauswertung unterzogen wird, in der als der wenigstens eine Vergleichsverlauf als dem Zielmaterial zugeordnet aus der Datenbank (10) abgerufen und/oder physikalische Eigenschaften beschreibende Ergebniswerte bestimmt werden, und
- das Vorhandensein des Zielmaterials unter Verwendung des gemäß der Ergebnisinformation ermittelten Übereinstimmungsgrades des wenigstens einen Vergleichsverlaufs mit dem Differenzsignal und/oder der bestimmten physikalischen Eigenschaften mit den physikalischen Eigenschaften des Zielmaterials detektiert wird.

7. Verfahren nach Anspruch 6 unter Rückbezug auf Anspruch 4 oder 5, **dadurch gekennzeichnet, dass** die Ergebnisse beider Vergleichsvorgänge zur gegenseitigen Plausibilisierung verwendet werden und/oder statistisch zusammengeführt werden, insbesondere unter stärkerer Gewichtung der Auswertung des Differenzsignals.

8. Verfahren nach einem der vorangehenden Ansprüche, **dadurch gekennzeichnet, dass** eine Voxelgröße von größer als 5 cm³, insbesondere größer als 15 cm³ verwendet wird.

9. Verfahren nach einem der vorangehenden Ansprüche, **dadurch gekennzeichnet, dass** die Detektionsinformation einen eine Anwesenheitswahrscheinlichkeit des Zielmaterials beschreibenden Wahrscheinlichkeitswert umfassend ermittelt wird.

10. Verfahren nach einem der vorangehenden Ansprüche, **dadurch gekennzeichnet, dass** als Zielmaterial ein Material eines Tumorgewebes oder koaguliertes Blut detektiert wird.

11. Magnetresonanzeinrichtung (1), aufweisend eine zur Ausführung eines Verfahrens nach einem der vorangehenden Ansprüche ausgebildete Steuereinrichtung (5).

12. Computerprogramm, welches die Schritte eines Verfahrens nach einem der Ansprüche 1 bis 10 durchführt, wenn es auf einer Steuereinrichtung (5) einer Magnetresonanzeinrichtung (1) ausgeführt wird.

13. Elektronisch lesbarer Datenträger, auf dem ein Computerprogramm nach Anspruch 12 gespeichert ist.

## Claims

1. Method for quantitative magnetic resonance imaging, wherein at least one time series of magnetic resonance images, which are assigned to different time instants after an excitation, of an examination region is recorded with a magnetic resonance device (1), wherein in the context of at least one finger printing evaluation from the time series for at least one part of the voxels of the magnetic resonance images, a temporal signal course is determined from the magnetic resonance data of all magnetic resonance images and in order to determine at least one result information the respective signal course is compared with at least one comparison course retrieved from a database (10),
**characterised in that**
in order to detect a target material, for which the anticipated portion is minimal compared with the portion of at least one other voxel material assumed in at least one voxel of interest in the magnetic resonance images,
- a first time series of magnetic resonance images is recorded without suppressing the signal of the target material and a second time series with suppression of the signal of the target material and
- by comparing the time series an item of detection information describing the presence of the target material is determined from at least one result information of at least one of the at least one finger printing evaluation for the at least one voxel of interest of the magnetic resonance images.

2. Method according to claim 1, **characterised in that** a suppression module adjusted to at least one physical property of the target material, in particular saturating the signal of the target material is used in order to suppress the signal of the target material of a used magnetic resonance sequence.

3. Method according to claim 2, **characterised in that** the suppression module comprises at least an inversion pulse and/or a binomial pulse sequence and/or is a T2-selective preparation module.

4. Method according to one of the preceding claims, **characterised in that** in order to compare the time series
- for both time series, a finger printing evaluation is carried out for the portions of candidate materials in the at least one voxel of interest as result information, wherein the candidate materials comprise the target material and the at least one voxel material, wherein comparison courses for all candidate materials are retrieved from the database (10), and its contribution to the signal course is determined as the respective portion,
- the result information of the two fingerprinting evaluations are compared with one another in order to determine the detection information.

5. Method according to claim 4, **characterised in that** within the scope of the fingerprinting evaluation, a subvoxel quantification is performed, and a lower value is recorded for the target material as a start value for the contribution than for the voxel materials.

6. Method according to one of the preceding claims, **characterised in that** in order to compare the time series:
- a differential signal is determined as the difference between the signal courses of the at least one voxel of interest,
- the differential signal as the signal course to be evaluated is subjected to a fingerprinting evaluation, in which as the at least one comparison course from the database (10) as assigned to the target material and/or result values describing physical properties are determined, and
- the existence of the target material is detected using the degree of similarity of the at least one comparison course determined according to the result information with the differential signal and/or the determined physical properties with the physical properties of the target material.

7. Method according to claim 6 with reference to claim 4 or 5, **characterised in that** the results of both comparison procedures are used for mutual plausibility and/or statistically merged, in particular using stronger weighting of the evaluation of the differential signal.

8. Method according to one of the preceding claims, **characterised in that** a voxel size of larger than 5 cm³, in particular larger than 15 cm³ is used.

9. Method according to one of the preceding claims, **characterised in that** the detection information is determined comprising a probability value describing a likelihood of the target material being present.

10. Method according to one of the preceding claims, **characterised in that** a material of a tumour tissue or coagulated blood is detected as the target material.

11. Magnetic resonance facility (1) having a control facility (5) embodied to execute a method according to one of the preceding claims.

12. Computer program, which carries out the steps of a method according to one of claims 1 to 10 if it is executed on a control facility (5) of a magnetic resonance facilty (1) .

13. Electronically readable data carrier, on which a computer program as claimed in claim 12 is stored.

## Revendications

1. Procédé d'imagerie quantitative par résonnance magnétique, dans lequel on enregistre, par un dispositif (1) de résonnance magnétique, une série temporelle d'images de résonnance magnétique, qui sont affectées à divers instants après une excitation, d'une partie à examiner, dans lequel, dans le cadre d'au moins une analyse d'empreinte digitale, on détermine à partir de la série temporelle, pour au moins une partie des voxels des images de résonnance magnétique, une courbe de signal en fonction du temps à partir des données de résonnance magnétique de toutes les images de résonnance magnétique et, pour la détermination d'au moins une information de résultat, on égalise la courbe respective de signal à au moins une courbe de comparaison appelée dans une base (10) de données,
**caractérisé en ce que**,
pour la détection d'un matériau cible, pour lequel la proportion escomptée, par rapport à la proportion d'au moins un autre matériau de voxel supposé dans au moins un voxel intéressant des images de résonnance magnétique est petite,
- on enregistre une première série temporelle d'images de résonnance magnétique sans suppression du signal du matériau cible et une deuxième série temporelle avec suppression du signal du matériau cible et,
- pour le au moins un voxel intéressant des images de résonnance magnétique, on détermine, par comparaison des séries temporelles, une information de détection décrivant la présence du matériau cible à partir d'au moins une information de résultat de la au moins l'une de au moins une analyse d'empreinte digitale.

2. Procédé suivant la revendication 1, **caractérisé en ce qu'**on utilise, pour la suppression du signal du matériau cible d'une séquence de résonnance magnétique utilisée, un module de suppression adapté à au moins une propriété physique du matériau cible, en particulier saturant le signal du matériau cible.

3. Procédé suivant la revendication 2, **caractérisé en ce que** le module de suppression comprend au moins une impulsion d'inversion et/ou un train d'impulsions binomial et/ou un module de préparation T2 sélectif.

4. Procédé suivant l'une des revendications précédentes, **caractérisé en ce que**, pour la comparaison des séries temporelles,
- on effectue pour les deux séries temporelles une analyse d'empreinte digitale pour les proportions de matériaux candidats dans le au moins un voxel intéressant comme information de résultat, dans lequel les matériaux candidats comprennent le matériau cible et le au moins un matériau de voxel, dans lequel on appelle des courbes de comparaison pour tous les matériaux candidats dans la base (10) de données et on détermine sa contribution à la courbe du signal comme la proportion respective,
- on compare les informations de résultat des deux analyses d'empreintes digitales entre elles pour la détermination de l'information de détection.

5. Procédé suivant la revendication 4, **caractérisé en ce que**, dans le cadre de l'analyse d'empreintes digitale, on effectue une quantification de sous-voxel et on ajoute pour le matériau cible comme valeur initiale pour la contribution une valeur plus petite que pour les matériaux de voxel.

6. Procédé suivant l'une des revendications précédentes, **caractérisé en ce que**, pour la comparaison des séries temporelles :
- on détermine un signal de différence comme différence des courbes du signal du au moins un voxel intéressant,
- on soumet le signal de différence comme courbe de signal à analyser à une analyse d'empreinte digitale, dans laquelle on appelle dans la base (10) de données aussi bien la au moins une courbe de comparaison que celle associée au matériau cible et/ou on détermine des valeurs de résultat décrivant des propriétés physiques, et
- on détecte la présence du matériau cible en utilisant le degré de coïncidence, déterminé suivant l'information de résultat, de la au moins une courbe de comparaison avec le signal de différence et/ou des propriétés physiques déterminées avec les propriétés physiques du matériau cible.

7. Procédé suivant la revendication 6 lorsqu'elle se rapporte à la revendication 4 ou 5, **caractérisé en ce que** l'on utilise le résultat des deux opérations de comparaison pour la vraisemblance mutuelle et/ou on les réunit statistiquement, en particulier en pondérant plus fortement l'analyse du signal de différence.

8. Procédé suivant l'une des revendications précédentes, **caractérisé en ce que** l'on utile une dimension de voxel de plus de 5 cm³, en particulier de plus de 15 cm³.

9. Procédé suivant l'une des revendications précédentes, **caractérisé en ce que** l'on détermine l'information de détection comprenant une valeur de probabilité décrivant une probabilité de présence du matériau cible.

10. Procédé suivant l'une des revendications précédentes, **caractérisé en ce que** l'on détecte comme matériau cible un matériau d'un tissu tumoral ou du sang coagulé.

11. Dispositif (1) de résonnance magnétique comportant un dispositif (5) de commande constitué pour l'exécution d'un procédé suivant l'une des revendications précédentes.

12. Programme d'ordinateur, qui effectue les stades d'un procédé suivant l'une des revendications 1 à 10 lorsqu'il est exécuté sur un dispositif (5) de commande d'un dispositif (1) de résonnance magnétique.

13. Support de données, déchiffrable électroniquement, sur lequel est mis en mémoire un programme d'ordinateur suivant la revendication 12.
